Europäisches Patentamt

European Patent Office

Office européen des brevets

(19)

(11) Publication number: **0 079 739**
**A2**

(12) **EUROPEAN PATENT APPLICATION**

(21) Application number: **82305926.6**

(22) Date of filing: **08.11.82**

(51) Int. Cl.³: **C 12 N 15/00**
**C 12 N 1/00, C 12 P 21/02**
**C 07 H 21/04, C 07 C 103/52**
**//C12R1/19, C12R1/865**

(30) Priority: **12.11.81 US 320632**

(43) Date of publication of application:
**25.05.83 Bulletin 83/21**

(84) Designated Contracting States:
**BE CH DE FR GB IT LI NL SE**

(71) Applicant: **THE UPJOHN COMPANY**
**301 Henrietta Street**
**Kalamazoo, Michigan 49001(US)**

(72) Inventor: **Dugaiczyk, Achilles**
**c/o The Upjohn Company 301 Henrietta Street**
**Kalamazoo Michigan 49001(US)**

(74) Representative: **Perry, Robert Edward et al,**
**GILL JENNINGS & EVERY 53-64 Chancery Lane**
**London WC2A 1HN(GB)**

(54) **Albumin-based nucleotides, their replication and use, and plasmids for use therein.**

(57) The DNA sequence coding for human serum albumin has been isolated and inserted as two fragments into two novel plasmids which can be replicated in *E. coli*. These novel fragments can be joined to provide a unitary DNA sequence which then can be cloned into a suitable host, e.g. *E. coli*, for the expression of human serum albumin (which is used extensively in medical practice in treating shock conditions).

EP 0 079 739 A2

## ALBUMIN-BASED NUCLEOTIDES, THEIR REPLICATION
## AND USE, AND PLASMIDS FOR USE THEREIN

This invention relates to nucleotides related to human serum albumin (HSA), their replication and use, and plasmids (and host substances) for use therein.

The gene for serum albumin is regulated in development. On the other hand, serum albumin is synthesised in mammals by the adult liver, and its plateau in adulthood. The embryonic liver and yolk sac, on the other hand, produce predominantly α-fetoprotein, but the synthesis decreases drastically after birth. Recently, Law et al determined the complete sequence of mouse α-fetoprotein mRNA, Nature 291 (1981) 201-205. The structure revealed extensive homology to mammalian serum albumin, indicating that the two proteins are encoded in the same gene family. Similar conclusions have been reached from studies on the α-fetoprotein genes of the rat and the mouse; see Jagodzinski et al, Proc. Natl. Acad. Sci. USA, 78 (1981) 3521-3525, and Gorin et al, J. Biol. Chem. 256 (1981) 1954-1959.

The complete nucleotide sequence of human serum mRNA has been determined from recombinant cDNA clones and from a primer-extended cDNA synthesis on the mRNA template. The sequence comprises 2,078 nucleotides, starting upstream of a potential ribosome binding site in the 5'-untranslated region. It contains all the translated codons and extends into the poly(A) at the 3'-terminus. Part of the translated sequence codes for a hydrophobic prepeptide met-lys-trp-val-thr-phe-ile-ser-leu-leu-phe-leu-phe-ser-ser-ala-tyr-ser, followed by a basic propeptide arg-gly-val-phe-arg-arg. These signal peptides are absent from mature serum albumin and, so far, have not been identified in their nascent state in humans. A remaining 1,755 nucleotides of the translated mRNA sequence code for 585 amino acids which are in agreement, with few exceptions, with the published amino acid data for human serum albumin. The mRNA sequence verifies and refines the repeating homology in the triple-domain structure of the serum albumin molecule.

~~DETAILED DESCRIPTION OF THE INVENTION~~

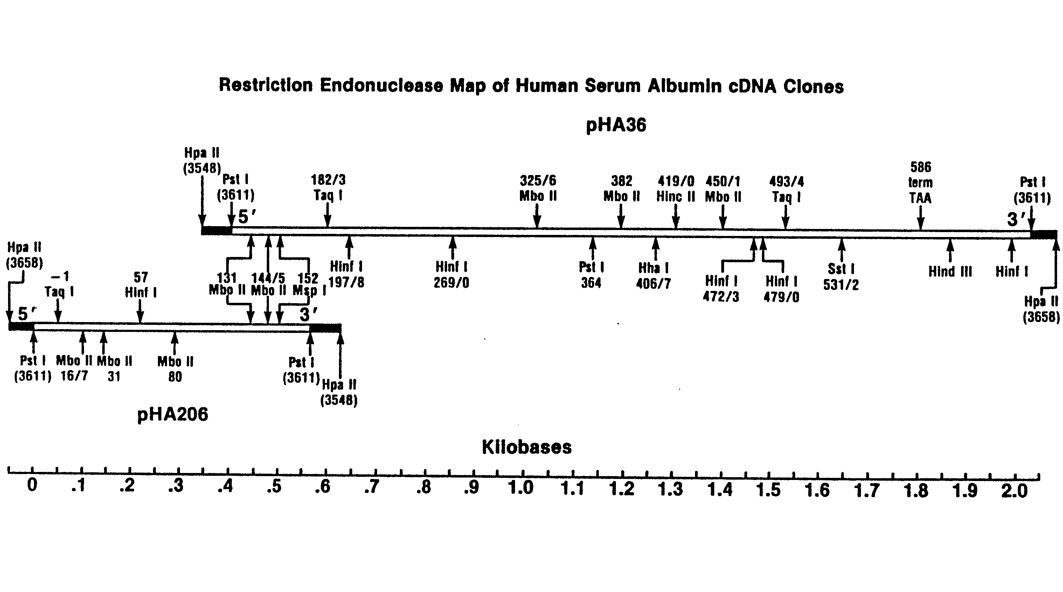

Human serum albumin cDNA is cloned into the PstI site of plasmid pBR322 by the oligo(dG)-oligo(dC) tailing technique. Plasmid DNA was isolated from 97 positive colonies which hybridized to the enriched albumin cDNA probe, and the recombinant plasmid pHA36 was found to contain the largest insert of an albumin cDNA sequence. Its restriction endonuclease map is shown in the drawing, together with a restriction map of the primer-extended plasmid clone pHA206. The latter was obtained in a second transformation experiment after initiating the cDNA synthesis from an internal primer. This primer was a 91 base pairs long DNA fragment, MspI(152)-TaqI(182/3), isolated from pHA36. The two plasmids, pHA36 and pHA206, share 0.15 kb of homologous DNA. Together, they encode the entire sequence for human serum albumin, starting with the CTT codon for leu -10 of the prepeptide and extending into the 3'-untranslated region of poly(A).

Sequence of the Albumin cDNA. The sequence was determined for the most part on both DNA strands to ensure accuracy. All of the restriction sites used to end-label DNA fragments were sequenced across by labeling a neighboring restriction site. The entire nucleotide sequence of the serum albumin mRNA, as determined from the cloned DNA in pHA36, pHA206, and from the primer-extended cDNA at the 5'-terminus of the message, is shown in the following Table 1. The inferred amino acid sequence is also indicated. The mRNA length is 2,078 nucleotides, of which 38 represent the 5'-untranslated region, 54 identify a prepeptide of 18 amino acids, 18 identify a propeptide of 6 amino acids, 1,755 code for the known 585 amino acids of serum albumin, 189 make up the 3'-untranslated region and 24 are the poly(A) sequence. Nucleotides 5 to 15 (-34 to -24) in the 5'-untranslated region (Table 1) are complementary to a 3'-terminal region of eukaryotic 18S RNA [Azad, A.A. and Deacon, N.J. (1980) Nucl. Acids Res. 8, 4365-4376] and thus could represent a ribosome binding site:

$$(5')...T\ T^C T\ C\ T\ T\ C\ T\ G\ T...............albumin\ mRNA$$
$$(3')...G\ A\ G\ G\ A\ A\ G\ G\ C\ G\ U\ C\ C\ m_2^6A\ m_2^6A.......18S\ RNA$$

The translated portion of the mRNA sequence codes for the signal peptide and the main body of the albumin polypeptide chain. The signal peptide is composed of a hydrophobic prepeptide of 18 amino acids and a basic propeptide of 6 amino acids (Table 1). Since pre-peptides are removed from nascent secretory proteins (like albumin) in the endoplasmic reticulum, they are seen only in vitro in heterologous translation systems. As yet, they have not been found within cells [Judah, J.D. and Quinn, P.S. (1977) FEBS 11th Mtg., Copenhagen 50, 21-29; and Strauss, A.W., Donohue, A.M., Bennett, C.D., Rodkey, J.A. and Alberts, A.W. (1977) Proc. Natl. Acad. Sci. USA 74, 1358-1362]. This is the first report of the presence and the sequence of a prepeptide for human serum albumin. As it is with other secretory proteins, the conversion of proalbumin to albumin takes place in the Golgi vesicles, and the enzyme responsible for this cleavage is probably cathepsin B [Judah, J.D. and Quinn, P.S. (1978) Nature 271, 384-385]. This is also a first report on the sequence of the propeptide for normal human serum albumin.

At the 3'-end of the message, the putative polyadenylation signal sequence, AATAAA, is located 164 nucleotides downstream from the amino acid termination codon TAA and 16 nucleotides upstream from the beginning of the poly(A) sequence. Another characteristic sequence located near the polyadenylation site has been identified by Benoist, et al. [Benoist, C., O'Hare, K., Breathnach, R. and Chambon, P. (1980) Nucl. Acids Res. 8, 127-142]; the concensus sequence from several mRNAs was concluded as TTTTCACTGC. A similar sequence, TTTTCTCTGT, is located 19 nucleotides upstream from the AATAAA hexanucleotide in the human albumin mRNA (Table 1).

TABLE 1

0079739

4083

-4-

```
                                          -18          p r o          -10
                                          Met lys trp val tlu phe Ile ser leu leu phe leu phe ser
                    GCTTTTCTCTTCTGTCAACCCCACAGCCCTTTGGCACA ATG AAG TGG GTA ACC TTT ATT TCC CTT CTT TTT CTC TTT AGC  (30)


        -1  -6     p r o    -1  1                              10                                    20
ser ala tyr ser arg gly val phe arg arg asp ala his lys ser glu val ala his arg phe lys asp leu gly glu glu asn phe lys
TCG GCT TAT TCC AGG GGT GTG TTT CGT CGA GAT GCA CAC AAG AGT GAG GTT GCT CAT CGG TTT AAA GAT TTG GGA GAA GAA AAT TTC AAA (170)


21                    30        34              40                                  50
ala leu val leu Ile ala phe ala gln tyr leu gln gln cys pro phe glu asp his val lys leu val asn glu val thr glu phe ala
GCC TTG GTG TTG ATT GCC TTT GCT CAG TAT CTT CAG CAG TGT CCA TTT GAA GAT CAT GTA AAA TTA GTG AAT GAA GTA ACT CAA TTT GCA (260)


51   53                  60      62                  70              75              80
lys thr cys val ala asp glu ser ala glu asn cys asp lys ser leu his thr leu phe gly asp lys leu cys thr val ala thr leu
AAA ACA TGT GTT GCT GAT GAG TCA GCT GAA AAT TGT GAC AAA TCA CTT CAT ACC CTT TTT CGA GAC AAA TTA TGC ACA GTT GCA ACT CTT (350)


81                      90 91                    100 101                      110
arg glu thr tyr gly glu met ala asp cys cys ala lys gln glu pro gly arg asn glu cys phe leu gln his lys asp asp asn pro
CGT GAA ACC TAT GGT GAA ATG GCT GAC TGC TGT GCA AAA CAA GAA CCT GGG AGA AAT GAA TGC TTC TTG CAA CAC AAA GAT GAC AAC CCA (440)


111                  120        124              130                              140
asn leu pro arg leu val arg pro glu val asp val met cys thr ala phe his asp asn glu glu thr phe leu lys lys tyr leu try
AAC CTC CCC CGA TTG GTG AGA CCA GAG GTT GAT GTG ATG TGC ACT GCT TTT CAT GAC AAT GAA GAG ACA TTT TTG AAA AAA TAC TTA TAT (330)


141                  150                    160              168 169 170
glu Ile ala arg arg his pro tyr phe tyr ala pro glu leu leu phe phe ala lys arg tyr lys ala ala phe thr glu cys cys gln
GAA ATT GCC AGA AGA CAT CCT TAC TTT TAT GCC CCG GAA CTC CTT TTC TTT GCT AAA AGG TAT AAA GCT GCT TTT ACA GAA TGT TGC CAA (620)


171              177        180                          190                      200
ala ala asp lys ala ala cys leu leu pro lys leu asp glu leu arg asp glu gly lys ala ser ser ala lys gln arg leu lys cys
GCT GCT GAT AAA GCT GCC TGC CTG TTG CCA AAG CTC GAT GAA CTT CGG GAT GAA GGG AAG GCT TCG TCT GCC AAA CAG AGA CTC AAG TGT (710)


201                  210                      220                      230
ala ser leu gln lys phe gly glu arg ala phe lys ala trp ala val ala arg leu ser gln arg phe pro lys ala glu phe ala glu
GCC AGT CTC CAA AAA TTT GGA GAA AGA GCT TTC AAA GCA TGG GCA GTA GCT CGC CTG AGC CAG AGA TTT CCC AAA GCT GAG TTT GCA GAA (300)
```

```
231                                      240                    245 246          250         253                        260
val ser lys leu val thr asp leu thr lys val his thr glu cys cys his gly asp leu leu glu cys ala asp asp arg ala asp leu
GTT TCC AAG TTA GTG ACA GAT CTT ACC AAA GTC CAC ACG GAA TGC TGC CAT GGA GAT CTG CTT GAA TGT GCT GAT GAC AGG GCG GAC CTT (890)


261           265              270                              278 279 280                           289 290
ala lys tyr Ile cys glu asn gln asp ser Ile ser ser lys leu lys glu cys cys glu lys pro leu leu glu lys ser his cys Ile
GCC AAG TAT ATC TGT GAA AAT CAA GAT TCG ATC TCC AGT AAA CTG AAG GAA TGC TGT GAA AAA CCT CTG TTG GAA AAA TCC CAC TGC ATT (980)


291                          300                              310                  316         320
ala glu val glu asn asp glu met pro ala asp leu pro ser leu ala ala asp phe val glu ser lys asp val cys lys asn tyr ala
GCC GAA GTG GAA AAT GAT GAG ATG CCT GCT GAC TTG CCT TCA TTA GCT GCT GAT TTT GTT GAA AGT AAG GAT GTT TGC AAA AAC TAT GCT (1070)


321                          330                              340                              350
glu ala lys asp val phe leu gly met phe leu tyr glu tyr ala arg arg his pro asp tyr ser val val leu leu leu arg leu ala
GAG GCA AAG GAT GTC TTC TTG GGC ATG TTT TTG TAT GAA TAT GCA AGA AGG CAT CCT GAT TAC TCT GTC GTG CTG CTG CTG AGA CTT GCC (1160)


351                          360 361                          369 370                          380
lys thr tyr glu thr thr leu glu lys cys cys ala ala ala asp pro his glu cys tyr ala lys val phe asp glu phe lys pro leu
AAG ACA TAT GAA ACC ACT CTA GAG AAG TGC TGT GCC GCT GCA GAT CCT CAT GAA TGC TAT GCC AAA GTG TTC GAT GAA TTT AAA CCT CCT (1250)


381                          390         392                      400                          410
val glu glu pro gln asn leu Ile lys gln asn cys glu leu phe glu gln leu gly glu tyr lys phe gln asn ala leu leu val arg
GTG GAA GAG CCT CAG AAT TTA ATC AAA CAA AAT TGT GAG CTT TTT GAG CAG CTT GGA GAG TAC AAA TTC CAG AAT GCG CTG TTA GTT CGT (1340)


411                          420                              430                  437 438      440
tyr thr lys lys val pro gln val ser thr pro thr leu val glu val ser arg asn leu gly lys val gly ser lys cys cys lys his
TAC ACC AAG AAA GTA CCC CAA GTG TCA ACT CCA ACT CTT GTA GAG GTC TCA AGA AAC CTA GGA AAA GTG GGC AGC AAA TGT TGT AAA CAT (1430)


441                          448     450                          460 461                      470
pro glu ala lys arg met pro cys ala glu asp tyr leu ser val val leu asn gln leu cys val leu his glu lys thr pro val ser
CCT GAA GCA AAA AGA ATG CCC TGT GCA GAA GAC TAT CTA TCC GTG GTC CTG AAC CAG TTA TGT GTG TTG CAT GAG AAA ACG CCA GTA AGT (1520)


471                      476 477      480                              490                          500
asp arg val thr lys cys cys thr glu ser leu val asn arg arg pro cys phe ser ala leu glu val asp glu thr tyr val pro lys
GAC AGA GTC ACC AAA TGC TGC ACA GAA TCC TTG GTG AAC AGG CGA CCA TGC TTT TCA GCT CTG GAA GTC GAT GAA ACA TAC GTT CCC AAA (1610)


501                          510         514                      520                          530
glu phe asn ala glu thr phe thr phe his ala asp Ile cys thr leu ser glu lys glu arg gln Ile lys lys gln thr ala leu val
GAG TTT AAT GCT GAA ACA TTC ACC TTC CAT GCA GAT ATA TGC ACA CTT TCT GAG AAG GAG AGA CAA ATC AAG AAA CAA ACT GCA CTT GTT (1700)
```

531                                    540                                    550                              558 559 560
glu leu val lys his lys pro lys ala thr lys glu gln leu lys ala val met asp asp phe ala ala phe val glu lys cys cys lys
GAG CTC GTG AAA CAC AAG CCC AAG GCA ACA AAA GAG CAA CTG AAA GCT GTT ATG GAT GAT TTC GCT GCT TTT GTA GAG AAG TGC TGC AAG (1790)

561                    567    570                                    580
ala asp asp lys glu thr cys phe ala glu glu gly lys lys leu val ala ala ser gln ala ala leu gly leu ter        ter
GCT GAC GAT AAG GAG ACC TGC TTT GCC GAG GAG GGT AAA AAA CTT GTT GCT GCA AGT CAA GCT GCC TTA GGC TTA TAA CATCACATTTAAAAG (1883)

                ter    ter
CATCTCAGCCTACCATGAGAATAAGAGAAAGAAAATGAAGATCAAAAGCTTATTCATCTGTTTTTCTTTTTCGTTGGTGTAAAGCCAACACCCTGTCTAAAAAAACATAAATTTCTTTAA (2002)

TCATTTTGCCTCTTTTCTCTGTGCTTCAATTAATAAAAAATGGAAAGAATCTAA..... 20 .....AA (2078)

-6-

0079739
4083

Following are examples which illustrate procedures~~, including the best mode,~~ for practicing the invention. These examples should not be construed as limiting. All percentages are by weight and all solvent mixture proportions are by volume unless otherwise noted.

## Example 1        Isolation of Messenger RNA

Human liver mRNA was obtained following the procedure of Chirgwin, et al [Chirgwin, J.M., Przybyla, A.E., MacDonald, R.J. and Rutter, W.J. (1979) Biochemistry 18, 5294-5299]. Immunoprecipitation of albumin containing polysomes was performed according to Taylor and Tse [Taylor, J.M. and Tse, T.P.H. (1976) J. Biol. Chem. 251, 7461-7467]. In vitro translation of mRNA was carried out in a reticulocyte cell-free system, following the instruction of the manufacturer (New England Nuclear). The translation products were separated electrophoretically according to Laemmli [Laemmli, J.K. (1970) Nature 227, 680-685.

## Example 2        Cloning Procedures

Double stranded cDNA was synthesized as described previously [Law, S., Tamaoki, T., Kreuzaler, F. and Dugaiczyk, A. (1980) Gene 10, 53-61]. It was annealed to PstI-linearized pBR322 DNA [Bolivar, F., Rodriguez, R.L., Greene, P.J., Betlach, M.C., Heyneker, H.L., Boyer, H.W., Crossa, J.H. and Falkow, S. (1977) Gene 2, 95-113] that had been tailed with 15 dG residues/3'-terminus [Dugaiczyk, A., Robberson, D.L. and Ullrich, A. (1980) Biochemistry 19, 5869-5873]. The annealed DNA was used to transform E. coli strain RR1, as detailed previously [Law, S., et al., Ibid.]. The albumin clones were selected using the colony hybridization method of Grunstein and Hogness [Grunstein, M. and Hogness, D.S. (1975) Proc. Natl. Acad. Sci. USA 72, 3961-3965], with [$^{32}$P]-labeled cDNA synthesized with the immunoprecipitated polysomal mRNA as template.

As shown in Example 5, plasmids pHA36 and pHA206 were deposited in E. coli HB101 hosts. The plasmids were obtained from E. coli RR1 hosts, described in this example, and transformed into E. coli HB101 by standard procedures well known to those of ordinary skill in this art. The E. coli RR1 hosts were lysed and then centrifuged to separate the chromosomal DNA, cell DNA and plasmid DNA. The plasmid DNA, remaining in the supernatant, is precipitated with ethanol and the precipitate is resuspended in buffer, e.g., TCM (10mM Tris·HCl, pH 8.0, 10 mM $CaCl_2$, 10 mM $MgCl_2$). The cells for transformation are prepared as follows: 120 ml of L-broth (1% tryptone, 0.5% yeast extract, 0.5% NaCl) are inoculated with an 18 hour culture of HB101 NRRL B-11371 and grown to an optical density of 0.6 at 600 nm. Cells are washed in cold 100 mM NaCl and resuspended for 15 minutes in 20 ml chilled 50 mM $CaCl_2$. Bacteria are then concentrated to one-tenth of this volume in $CaCl_2$ and mixed 2:1 (v:v) with annealed plasmid DNA, prepared as described above. After chilling the cell-DNA mixture for 15 minutes, it is heat shocked at 42°C for 2 minutes, then allowed to equilibrate at room temperature for ten minutes before addition of L-broth 10 times the volume of the cell-DNA suspension. Transformed cells are incubated in broth at 37°C for one hour before inoculating selective media (L-agar plus 10 µg/ml tetracycline) with 200 µl/plate. Plates are incubated at 37°C for 48 hours to allow the growth of transformants.

Example 3    Mapping of Restriction Endonuclease Sites

Restriction endonucleases were obtained from Bethesda Research Laboratories and New England Biolabs and were used according to the manufacturers' instructions. The digested DNA fragments were analyzed electrophoretically on agarose [Helling, R.B., Goodman, H.M. and Boyer, H.W. (1974) J. Virol. 14, 1235-1244] or acrylamide [Dingman, C., Fisher, M.P. and Kakefuda, T. (1972) Biochemistry 11, 1242-1250] gels.

Example 4    DNA Sequencing

DNA fragments were dephosphorylated with bacterial alkaline phosphatase (Worthington) and labeled at the 5'-ends with polynucleotide kinase (Boehringer-Mannheim) and $\gamma[^{32}P]ATP$. Following digestion with a second restriction endonuclease and electrophoretic separation of the fragments, DNA sequence determination was done according to the procedure of Maxam and Gilbert [Maxam, A. and Gilbert, W. (1980) Methods Enzym. 65, 499-560] and the degradation products were separated electrophoretically on 0.4 mm acrylamide gels as described by Sanger and Coulson [Sanger, F. and Coulson, R. (1978) FEBS Letters 87, 107-110].

Example 5    Recombinant Plasmids pHA36 and pHA206

As disclosed in Example 2, albumin clones were selected by hybridizing to the enriched albumin cDNA probe. Plasmid pHA36 contained the largest insert of an albumin cDNA sequence. Both plasmids pHA36 and pHA206 have been deposited in a viable E. coli host in the permanent collection of the Northern Regional Research Laboratory (NRRL), U.S. Department of Agriculture, Peoria, Illinois, U.S.A. Their accession numbers in this repository are as follows:

HB101(pHA36) - NRRL B-12551

HB101(pHA206) - NRRL B-12550

E. coli HB101 is a known and widely available host microbe. Its NRRL accession number is NRRL B-11371.

NRRL B-12550 and NRRL B-12551 are available to the public. ~~upon the grant of a patent. It should be understood that the availability~~ of these deposits does not constitute a license to practice the subject invention in derogation of patent rights granted with the subject ~~instrument by governmental action.~~

E. coli RR1 and E. coli HB101 are known and widely available host microbes. Their NRRL accession numbers are NRRL B-12186 and NRRL B-11371, respectively.

pBR322 is a well known and widely available plasmid. It can be obtained from the following host deposit by standard procedures:

NRRL B-12014 - E. coli RR1 (pBR322).

YEp6 is a well known and widely available yeast episomal plasmid. It can be obtained from the following host deposit by standard procedures:

E. coli HB101 (YEp6) - NRRL B-12093.

Example 6      Assembly of the Serum Albumin Gene

Assembling the pieces together is a straighforward task of restriction enzymology. There is only one MspI site in the overlapping DNA sequence of the two cDNA clones. Two enzymatic steps of (i) MspI digestion of the two DNAs, followed by (ii) the use of ligase, an enzyme that seals DNA fragments, will give the desired product. Although two other undesired DNA species will also be obtained in the course of this recombination reaction, both of them will differ substantially in size. Thus, separation and isolation of the desired DNA species will be achieved.

The assembled DNA clone can be used to transform two types of cells:

(a)  Escherichia coli

(b)  Saccharomyces cerevisiae

(a)  The vector of choice is plasmid pBR322, the same that has been successfully used for cloning of the two fragmented pieces of the serum albumin cDNA.

(b)   In order to transform yeast with the serum albumin structural gene sequence, the DNA must be inserted into one of the existing yeast plasmid vectors.  This can be accomplished by taking advantage of the fact that several restriction endonuclease recognition sequences are absent from the cloned serum albumin DNA.  Synthetic EcoRl DNA linkers can be ligated to the DNA fragment containing the serum albumin sequence followed by insertion (ligation) into one of the yeast plasmid vectors, e.g., YEp6, at the Eco Rl cloning site. The fused chimeric plasmid can be used to transform yeast according to an established procedure [Hinnen, A., Hicks, J.B. and Fink, G.R. (1978) Proc. Natl. Acad. Sci. USA, 75, 1929].  YEp6 can be obtained from the NRRL repository, as disclosed supra.

Example 7       Expression of the Serum Albumin Gene

The main body of the structural gene will be transcribed by the E. coli or yeast enzymes.  If little or no albumin is produced with the selected host, then an Escherichia coli promoter DNA sequence carrying an initiation codon, i.e., ATG, can be ligated at the beginning of the serum albumin structural gene.  Such elements are known and available, e.g., lac promoter used for the expression of human interferon gene in E. coli [Proc. Natl. Acad. Sci. 77, 5230 (1980)]; source of promoter DNA [Proc. Natl. Acad. Sci. 76, 760 (1979)].  Also, see Nature, Vol. 281, October 18, 1979.  It has already been documented that such Escherichia coli promoter sequences function well in the expression of foreign genes in Escherichia coli [Mercereau-Puijalon, O., Royal, A., Cami, B., Garapin, A., Krust, A., Gannon, I. and Kourilsky, P. (1978) Nature 275, 505; and Goeddel, D.V., Kleid, D.G., Bolivar, F., Heyneker, H.L., Yansura, D.G., Grea, R., Hirose, T., Kraszewski, A., Itakura, K., and Riggs, A. (1979) Natl. Acad. Sci. USA 76, 106]. For expression in yeast, see Rose, M., Casadaban, M.J. and Botstein, D. (1981) Proc. Natl. Acad. Sci. USA 78, 2460 and 4466.

Example 8       Screening of Clones Producing Albumin

Immunological methods can be used to detect small amounts of albumin made in a bacterium.  Flat disks of flexible polyvinyl are coated with the IgG fraction from an immune serum and the disks are pressed onto an agar plate so that antigen released from an in situ lysed microbial colony can bind to the fixed antibody.  The plastic

**0079739**

4083

disk is then incubated with the same total IgG fraction labeled with radioactive iodine so that other determinants on the bound antigen can in turn bind the iodinated antibody. Radioactive areas on the disk expose X-ray film during autoradiography and thus identify colonies producing the protein which is being screened for. Detailed protocols of this procedure have been published [Broome, S. and Gilbert, W. (1978) Proc. Natl. Acad. Sci. USA, 75, 2746]. The purification of human serum albumin can be accomplished by using procedures well known in the art. For example, procedures disclosed in a chapter by T. Peters: Purification and Properties of Serum Albumin, in: The Plasma Proteins, Putnam, Ed. Academic Press, New York, 1975, can be used.

The work described herein was all done in conformity with physical and biological containment requirements specified in the NIH Guidelines.

## CLAIMS

1. Plasmid pHA36, having a restriction endonuclease pattern as shown in the drawing.

2. Plasmid pHA206, having a restriction endonuclease pattern as shown in the drawing.

3. E. coli HB101 (pHA36) having the deposit accession number NRRL B-12551.

4. E. coli HB101 (pHA206) having the deposit accession number NRRL B-12550.

5. A microorganism modified to contain a nucleotide sequence coding for the amino acid sequence of human serum albumin; said nucleotide sequence is as follows:

```
                                                              -10
                                                    leu phe leu phe ser
                                                    CTT TTT CTC TTT AGC   (30)


          -1  -6  pro        -1   1                    10                          20
         ser ala tyr ser arg gly val phe arg arg asp ala his lys ser glu val ala his lys arg phe lys asp leu gly glu glu asn phe lys
         TCG GCT TAT TCC AGG GGT GTG TTT CGT CGA GAT GCA CAC AAG AGT GAG GTT GCT CAT CGG TTT AAA GAT TTG GGA GAA GAA AAT TTC AAA   (170)


          21                        30          34                    40                          50
         ala leu val leu ile ala phe ala gln tyr leu gln gln cys pro phe gln asp his val lys leu val asn glu val thr glu phe ala
         GCC TTG GTG TTG ATT GCC TTT GCT CAG TAT CTT CAG CAG TGT CCT TTT CAG GAT CAT GTA AAA TTA GTG AAT GAA GTA ACT GAA TTT GCA   (260)


          51  53                    60  62                  70          75                80
         lys thr cys val ala asp glu ser ala asp lys ser his thr leu cys asp lys ser leu his thr val ala thr leu
         AAA ACA TGT GTT GCT GAT GAG TCA GCT GAC AAA TCA CAT ACC CTT TGC GAC AAA TCA CTT CAT ACC GTT GCA ACT CTT   (350)


          81                        90  91                  100 101                      110
         arg glu thr tyr gly glu met ala asp cys cys ala lys gln glu pro gly arg asn asn glu gln glu thr phe his asp asn asn pro
         CGT GAA ACC TAT GGT GAA ATG GCT GAT TGT TGC GCA AAA CAA GAA CCT GGG AGA AAT AAT GAA CAA GAA ACA TTT CAT GAC AAC AAC CCA   (440)


          111                       120 124                 130                         140
         asn leu pro arg leu val arg pro glu val asp val met cys thr ala phe his asp asn glu glu thr phe leu lys tyr lys ala
         AAC CTC CCC CGA TTG GTG AGA CCA GAG GTT GAT GTG ATG TGC ACT GCT TTT CAT GAC AAC


          141                       150                     160                 168 169 170
         glu ile ala arg arg his pro tyr phe tyr ala pro glu leu leu phe phe ala lys            lys lys tyr leu try
         GAA ATT GCC AGA AGA CAT CCT TAC TTT TAT GCC CCG GAA CTC CTT TTC TTT GCT AAA            AAA AAA TAC TTA TAT   (330)


                                                                          cys cys gln
                                                                          TGC TGC CAA   (620)


          171                       177 180                 190                         200
         ala ala asp lys ala ala cys leu leu pro lys leu asp glu gly leu arg asp glu gly gly ala ser ser ala lys ser ala lys cys
         GCT GCT GAT AAA GCT GCC TGC CTG CTT CCA AAG CTC GAT GAA GGG CTT CGG GAT GAA CTT CGC CGC GAA AAG CAG AAG CTC AAG TGT   (710)


          201                       210                     220                         230
         ala ser leu gln lys phe gly glu glu arg            val ala arg leu ser gln arg phe pro lys ala glu gln glu
         GCC AGT CTC CAA AAA TTT GGA GAA GAA AGA            GCA GTA GCT CGC CTG AGC CAG CAG AGA TTT CCC AAA GCT GAG GCA GAA   (300)
```

231          240    245 246   250  253     260
val ser lys leu val thr asp leu thr lys val his thr glu cys cys his gly asp leu leu glu cys ala asp asp arg ala asp leu
GTT TCC AAG TTA GTG ACA GAT CTT ACC AAA GTC CAC ACG GAA TGC TGC CAT GGA GAT CTG CTT GAA TGT GCT GAT GAC AGG GCG GAC CTT (890)

261    265     270       278 279 280       289 290
ala lys tyr ile cys glu asn gln asp ser ile ser ser lys leu lys glu cys cys glu lys pro leu leu glu lys ser his cys ile
GCC AAG TAT ATC TGT GAA AAT CAA GAT TCG ATC TCC AGT AAA CTG AAG GAA TGC TGT GAA AAA CCT CTG TTG GAA AAA TCC CAC TGC ATT (980)

291         300       310   316   320
ala glu val glu asn asp glu met pro ala asp leu pro ser leu ala ala asp phe val glu ser lys asp val cys lys asn tyr ala
GCC GAA GTG GAA AAT GAT GAG ATG CCT GCT GAC TTG CCT TCA TTA GCT GCT GAT TTT GTT GAA AGT AAG GAT GTT TGC AAA AAC TAT GCT (1070)

321         330       340       350
glu ala lys asp val phe leu gly met phe leu tyr glu tyr ala arg arg his pro asp tyr ser val val leu leu leu arg leu ala
GAG GCA AAG GAT GTC TTC TTG GGC ATG TTT TTG TAT GAA TAT GCA AGA AGG CAT CCT GAT TAC TCT GTC GTG CTG CTG CTG AGA CTT GCC (1160)

351        360 361      369 370      380
lys thr tyr glu thr thr leu glu lys cys cys ala ala ala asp pro his glu cys tyr ala lys val phe asp glu phe lys pro leu
AAG ACA TAT GAA ACC ACT CTA GAG AAG TGC TGT GCC GCT GCA GAT CCT CAT GAA TGC TAT GCC AAA GTG TTC GAT GAA TTT AAA CCT CCT (1250)

381       390  392      400      410
val glu glu pro gln asn leu ile lys gln asn cys glu leu phe glu gln leu gly glu tyr lys phe gln asn ala leu leu val arg
GTG GAA GAG CCT CAG AAT TTA ATC AAA CAA AAT TGT GAG CTT TTT GAG CAG CTT GGA GAG TAC AAA TTC CAG AAT GCG CTG TTA GTT CGT (1340)

411       420       430    437 438  440
tyr thr lys lys val pro gln val ser thr pro thr leu val glu val ser arg asn leu gly lys val gly ser lys cys cys lys his
TAC ACC AAG AAA GTA CCC CAA GTG TCA ACT CCA ACT CTT GTA GAG GTC TCA AGA AAC CTA GGA AAA GTG GGC AGC AAA TGT TGT AAA CAT (1430)

441     448  450       460 461     470
pro glu ala lys arg met pro cys ala glu asp tyr leu ser val val leu asn gln leu cys val leu his glu lys thr pro val ser
CCT GAA GCA AAA AGA ATG CCC TGT GCA GAA GAC TAT CTA TCC GTG GTC CTG AAC CAG TTA TGT GTG TTG CAT GAG AAA ACG CCA GTA AGT (1520)

471    476 477  480       490      500
asp arg val thr lys cys cys thr glu ser leu val asn arg arg pro cys phe ser ala leu glu val asp glu thr tyr val pro lys
GAC AGA GTC ACC AAA TGC TGC ACA GAA TCC TTG GTG AAC AGG CGA CCA TGC TTT TCA GCT CTG GAA GTC GAT GAA ACA TAC GTT CCC AAA (1610)

501       510  514     520     530
glu phe asn ala glu thr phe thr phe his ala asp ile cys thr leu ser glu lys glu arg gln ile lys lys gln thr ala leu val
GAG TTT AAT GCT GAA ACA TTC ACC TTC CAT GCA GAT ATA TGC ACA CTT TCT GAG AAG GAG AGA CAA ATC AAG AAA CAA ACT GCA CTT GTT (1700)

531 540 550 558 559 560
glu leu val lys his lys pro lys ala thr lys glu gln leu lys ala val met asp asp phe ala ala phe val glu lys cys cys lys
GAG CTC GTG AAA CAC AAG CCC AAG GCA ACA AAA GAG CAA CTG AAA GCT GTT ATG GAT GAT TTC GCT GCT TTT GTA GAG AAG TGC TGC AAG (1790)

561 567 570 580
ala asp asp lys glu thr cys phe ala glu glu gly lys lys leu val ala ala ser gln ala ala leu gly leu ter     ter
GCT GAC GAT AAG GAG ACC TGC TTT GCC GAG GAG GGT AAA AAA CTT GTT GCT GCA AGT CAA GCT GCC TTA GGC TTA TAA CATCACATTTAAAAG (1883)

          ter    ter
CATCTCAGCCTACCATGAGAATAAGAGAAAGAAAATGAAGATCAAAAGCTTATTCATCTGTTTTTCTTTTTCGTTGGTGTAAAGCCAACACCCTGTCTAAAAAACATAAATTTCTTTAA (2002)

TCATTTTGCCTCTTTTCTCTGTGCTTCAATTAATAAAAAAATGGAAAGAATCTAA..... 20 .....AA (2078)

-15-

6. Nucleotide sequence of the cDNA of human serum albumin, said nucleotide sequence is as follows:

```
                      10                            20
  1   asp ala his lys ser glu val ala his arg phe lys asp leu gly glu glu asn phe lys
      GAT GCA CAC AAG AGT GAG GTT GCT CAT CGG TTT AAA GAT CTG GGA GAA GAA AAT TTC AAA   (170)

              30      34              40                          50
 21   ala leu val leu ile ala phe ala gln tyr leu gln gln cys pro phe glu asp his val lys leu val asn glu val thr glu phe ala
      GCC TTG GTG CTG ATT GCC TTT GCC CAG TAT CTT CAG CAG TGT CCA TTT GAA GAT CAT GTA AAA TTA GTG AAT GAA GTA ACT GAA TTT GCA   (260)

      53          60      62              70          75          80
 51   lys thr cys val ala asp glu ser ala glu asn cys asp lys ser leu his thr leu phe gly asp lys leu cys thr val ala thr leu
      AAA ACA TGT GTT GCT GAT GAG TCA GCT GAA AAT TGC GAC AAA TCA CTT CAT ACC CTT TTT GGA GAC AAA TTA TGC ACA GTT GCA ACT CTT   (350)

              90  91                      100 101                      110
 81   arg glu thr tyr gly glu met ala asp cys cys ala lys gln glu pro glu arg asn glu cys phe leu gln his lys asp asp asn pro
      CGT GAA ACC TAT GGT GAA ATG GCT GAC TGC TGT GCA AAA CAA GAA CCT GAA AGA AAT GAA TGC TTC TTG CAA CAC AAA GAT GAC AAC CCA   (440)

                      120     124             130
111   asn leu pro arg leu val arg pro glu val asp val met cys thr ala phe his asp asn glu glu thr phe leu lys lys tyr leu tyr
      AAC CTC CCC CGA CTC GTG AGA CCA GAG GTT GAT GTG ATG TGC ACT GCT TTT CAT GAC AAT GAA GAG ACA TTT TTG AAA AAA TAC TTA TAT   (530)

                      150                     160         168 169 170
141   glu ile ala arg arg his pro tyr phe tyr ala pro glu leu leu phe phe ala lys arg tyr lys ala ala phe thr glu cys cys gln
      GAA ATT GCC AGA AGA CAT CCT TAT TTT TAT GCC CCA GAA CTC CTT TTC TTT GCT AAA AGG TAT AAA GCT GCT TTT ACA GAA TGT TGC CAA   (620)

      177         180                     190                     200
171   ala ala asp lys ala ala cys leu leu pro lys leu asp glu leu arg asp glu gly lys ala ser ser ala lys gln arg leu lys cys
      GCT GCT GAT AAA GCT GCC TGC CTG TTG CCA AAG CTC GAT GAA CTC CGG GAT GAA GGG AAG GCT TCG TCT GCC AAA CAG AGA CTC AAG TGT   (710)

                      210                     220                     230
201   ala ser leu gln lys phe gly glu arg ala phe lys ala trp ala val ala arg leu ser gln arg phe pro lys ala glu phe ala glu
      GCC AGT CTC CAA AAA TTT GGA GAA AGA GCT TTC AAA GCA TGG GCA GTA GCT CGC CTG AGC CAG AGA TTT CCC AAA GCT GAG TTT GCA GAA   (800)
```

231

val ser lys leu val thr asp leu thr lys val his thr glu cys cys his gly asp leu leu glu cys ala asp asp arg ala asp leu
GTT TCC AAG TTA GTG ACA GAT CTT ACC AAA GTC CAC ACG GAA TGC TGC CAT GGA GAT CTG CTT GAA TGT GCT GAT GAC AGG GCG GAC CTT (890)

261 265 270 278 279 280 289 290

ala lys tyr ile cys glu asn gln asp ser ile ser ser lys leu lys glu cys cys glu lys pro leu leu glu lys ser his cys ile
GCC AAG TAT ATC TGT GAA AAT CAA GAT TCG ATC TCC AGT AAA CTG AAG GAA TGC TGT GAA AAA CCT CTG TTG GAA AAA TCC CAC TGC ATT (980)

291 300 310 316 320

ala glu val glu asn asp glu met pro ala asp leu pro ser leu ala ala asp phe val glu ser lys asp val cys lys asn tyr ala
GCC GAA GTG GAA AAT GAT GAG ATG CCT GCT GAC TTG CCT TCA TTA GCT GCT GAT TTT GTT GAA AGT AAG GAT GTT TGC AAA AAC TAT GCT (1070)

321 330 340 350

glu ala lys asp val phe leu gly met phe leu tyr glu tyr ala arg arg his pro asp tyr ser val val leu leu leu arg leu ala
GAG GCA AAG GAT GTC TTC TTG GGC ATG TTT TTG TAT GAA TAT GCA AGA AGG CAT CCT GAT TAC TCT GTC GTG CTG CTG CTG AGA CTT GCC (1160)

351 360 361 369 370 380

lys thr tyr glu thr thr leu glu lys cys cys ala ala ala asp pro his glu cys tyr ala lys val phe asp glu phe lys pro leu
AAG ACA TAT GAA ACC ACT CTA GAG AAG TGC TGT GCC GCT GCA GAT CCT CAT GAA TGC TAT GCC AAA GTG TTC GAT GAA TTT AAA CCT CTT (1250)

381 390 392 400 410

val glu glu pro gln asn leu ile lys gln asn cys glu leu phe glu gln leu gly glu tyr lys phe gln asn ala leu leu val arg
GTG GAA GAG CCT CAG AAT TTA ATC AAA CAA AAT TGT GAG CTT TTT GAG CAG CTT GGA GAG TAC AAA TTC CAG AAT GCG CTG TTA GTT CGT (1340)

411 420 430 437 438 440

tyr thr lys lys val pro gln val ser thr pro thr leu val glu val ser arg asn leu gly lys val gly ser lys cys cys lys his
TAC ACC AAG AAA GTA CCC CAA GTG TCA ACT CCA ACT CTT GTA GAG GTC TCA AGA AAC CTA GGA AAA GTG GGC AGC AAA TGT TGT AAA CAT (1430)

441 448 450 460 461 470

pro glu ala lys arg met pro cys ala glu asp tyr leu ser val val leu asn gln leu cys val leu his glu lys thr pro val ser
CCT GAA GCA AAA AGA ATG CCC TGT GCA GAA GAC TAT CTA TCC GTG GTC CTG AAC CAG TTA TGT GTG TTG CAT GAG AAA ACG CCA GTA AGT (1520)

471 476 477 480 490 500

asp arg val thr lys cys cys thr glu ser leu val asn arg arg pro cys phe ser ala leu glu val asp glu thr tyr val pro lys
GAC AGA GTC ACC AAA TGC TGC ACA GAA TCC TTG GTG AAC AGG CGA CCA TGC TTT TCA GCT CTG GAA GTC GAT GAA ACA TAC GTT CCC AAA (1610)

501 510 514 520 530

glu phe asn ala glu thr phe thr phe his ala asp ile cys thr leu ser glu lys glu arg gln ile lys lys gln thr ala leu val
GAG TTT AAT GCT GAA ACA TTC ACC TTC CAT GCA GAT ATA TGC ACA CTT TCT GAG AAG GAG AGA CAA ATC AAG AAA CAA ACT GCA CTT GTT (1700)

531
glu leu val lys his lys pro lys ala thr lys glu gln leu lys ala val met asp asp phe ala ala phe val glu lys cys cys lys
GAG CTC GTG AAA CAC AAG CCC AAG GCA ACA AAA GAG CAA CTG AAA GCT GTT ATG GAT GAT TTC GCT GCT TTT GTA GAG AAG TGC TGC AAG (1790)

561
ala asp asp lys glu thr cys phe ala glu glu gly lys lys leu val ala ala ser gln ala ala leu gly leu ter       ter
GCT GAC GAT AAG GAG ACC TGC TTT GCC GAG GAG GGT AAA AAA CTT GTT GCT GCA AGT CAA GCT GCC TTA GGC TTA TAA CATCACATTTAAAAG (1883)

ter   ter
CATCTCAGCCTACCATGAGAATAAGAGAAAGAAAATGAAGATCAAAAGCTTATTCATCTGTTTTTCTTTTTCGTTGGTGTAAAGCCAACACCCTGTCTAAAAAAACATAAATTTCTTTAA (2002)

TCATTTTGCCTCTTTTCTCTGTGCTTCAATTAATAAAAAAATGGAAAGAATCTAA..... 20 .....AA (2078)

0079739
4083

7. Nucleotide sequence coding for the prepeptide of human serum albumin, said nucleotide sequence is as follows:

```
                                             -18                                                      -10
                                             Met lys trp val tlu phe ile ser leu leu phe leu phe ser
                                                              p   r   o
GCTTTCTCTTCTGTCAACCCCACAGCCCTTGGCACA ATG AAG TGG GTA ACC TTT ATT TCC CTT CTT TTT CTC TTT AGC (30)

        -6              p   r   o           -1
ser ala tyr ser arg gly val phe arg arg
TCG GCT TAT TCC AGG GGT GTG TTT CGT CGA
```

-20-

8. Nucleotide sequence coding for pro human serum albumin, said nucleotide sequence is as follows:

```
        35      30      25      20      15      10      5

                        -6          p r o  -1   1                                        10                          20
        arg gly val phe arg arg asp ala his lys ser glu val ala his arg phe lys asp leu gly glu glu asn phe lys
        AGG GGT GTG TTT CGT CGA GAT GCA CAC AAG AGT GAG GTT GCT CAT CGG TTT AAA GAT TTG GGA GAA GAA AAT TTC AAA  (170)

 21                                          30                          40                          50
 ala leu val leu ile ala phe ala gln tyr leu gln gln cys pro phe glu asp his val lys leu val asn glu val thr glu phe ala
 GCC TTG GTG TTG ATT GCC TTT CAG TAT CTT CAG CAG TGT CCA TTT GAA GAT CAT GTA AAA TTA GTG AAT GAA GTA ACT GAA TTT GCA  (260)

 51      53                          60      62          70          75          80
 lys thr cys val ala ala asp glu ser ala glu asn cys asp lys ser leu his thr leu phe gly asp lys leu cys thr val ala thr leu
 AAA ACA TGT GTT GCT GAT GAG TCA GCT GAA AAT TGT GAC AAA TCA CTT CAT ACC CTT TTC GGA GAC AAA TTA TGC ACA GTT GCA ACT CTT  (350)

 81                          90  91          100 101                 110
 arg glu thr tyr gly glu met ala asp cys cys ala lys gln glu pro glu arg asn glu cys phe leu gln his lys asp asp asn pro
 CGT GAA ACC TAT GGT GAA ATG GCT GAC TGC TGT GCA AAA CAA GAA CCT GGG GAA AGA AAT GAA TGC TTC TTG CAA CAC AAA GAC GAC AAC CCA  (440)

111                                 120 124                 130                         140
asn leu pro arg leu val arg pro glu val asp val met cys thr ala phe his asp asn glu glu thr phe leu lys lys tyr leu tyr
AAC CTC CCC CGA TTG GTG AGA CCA GAG GTT GAT GTG ATG TGC ACT GCT TTT CAT GAC AAT GAG GAG ACA TTT TTG AAA AAA TAC TTA TAT  (530)

141                                         150                 160             168 169 170
glu ile ala arg arg his pro tyr phe tyr ala pro glu leu leu phe phe ala lys arg tyr lys ala ala phe thr glu cys cys gln
GAA ATT GCC AGA AGA CAT CCT TAC TTT TAT GCC CCG GAA CTC CTT TTC TTT GCT AAA AGG TAT AAA GCT GCT TTT ACA GAA TGT TGC CAA  (620)

171                             177     180                         190                         200
ala ala asp lys ala ala cys leu leu pro lys leu asp glu leu arg asp glu gly lys ala ser ser ala lys gln arg leu lys cys
GCT GCT GAT AAA GCT GCC TGC CTG TTG CCA AAG CTC GAT GAA CTT CGG GAT GAA GGG AAG GCT TCG TCT GCC AAA CAG AGA CTC AAG TGT  (710)

201                             210                 220                         230
ala ser leu gln lys phe gly glu arg ala phe lys ala trp ala val ala arg leu ser gln arg phe pro lys ala glu phe ala glu
GCC AGT CTC CAA AAA TTT GGA GAA AGA GCT TTC AAA GCA TGG GCA GTA GCT CGC CTG AGC CAG AGA TTT CCC AAA GCT GAG TTT GCA GAA  (300)
```

231                             240                     245 246           250          253                  260

val ser lys leu val thr asp leu thr lys val his thr glu cys cys his gly asp leu leu glu cys ala asp asp arg ala asp leu

GTT TCC AAG TTA GTG ACA GAT CTT ACC AAA GTC CAC ACG GAA TGC TGC CAT GGA GAT CTG CTT GAA TGT GCT GAT GAC AGG GCG GAC CTT (890)

261          265                  270                          278 279 280                     289 290

ala lys tyr ile cys glu asn gln asp ser ile ser ser lys leu lys glu cys cys glu lys pro leu leu glu lys ser his cys ile

GCC AAG TAT ATC TGT GAA AAT CAA GAT TCG ATC TCC AGT AAA CTG AAG GAA TGC TGT GAA AAA CCT CTG TTG GAA AAA TCC CAC TGC ATT (980)

291                             300                                310             316             320

ala glu val glu asn asp glu met pro ala asp leu pro ser leu ala ala asp phe val glu ser lys asp val cys lys asn tyr ala

GCC GAA GTG GAA AAT GAT GAG ATG CCT GCT GAC TTG CCT TCA TTA GCT GCT GAT TTT GTT GAA AGT AAG GAT GTT TGC AAA AAC TAT GCT (1070)

321                             330                                340                           350

glu ala lys asp val phe leu gly met phe leu tyr glu tyr ala arg arg his pro asp tyr ser val val leu leu leu arg leu ala

GAG GCA AAG GAT GTC TTC TTG GGC ATG TTT TTG TAT GAA TAT GCA AGA AGG CAT CCT GAT TAC TCT GTC GTG CTG CTG CTG AGA CTT GCC (1160)

351                             360 361                            369 370                         380

lys thr tyr glu thr thr leu glu lys cys cys ala ala ala asp pro his glu cys tyr ala lys val phe asp glu phe lys pro leu

AAG ACA TAT GAA ACC ACT CTA GAG AAG TGC TGT GCC GCT GCA GAT CCT CAT GAA TGC TAT GCC AAA GTG TTC GAT GAA TTT AAA CCT CCT (1250)

381                             390     392                             400                           410

val glu glu pro gln asn leu ile lys gln asn cys glu leu phe glu gln leu gly glu tyr lys phe gln asn ala leu leu val arg

GTG GAA GAG CCT CAG AAT TTA ATC AAA CAA AAT TGT GAG CTT TTT GAG CAG CTT GGA GAG TAC AAA TTC CAG AAT GCG CTG TTA GTT CGT (1340)

411                             420                                430                     437 438     440

tyr thr lys lys val pro gln val ser thr pro thr leu val glu val ser arg asn leu gly lys val gly ser lys cys cys lys his

TAC ACC AAG AAA GTA CCC CAA GTG TCA ACT CCA ACT CTT GTA GAG GTC TCA AGA AAC CTA GGA AAA GTG GGC AGC AAA TGT TGT AAA CAT (1430)

441                      448     450                                460 461                        470

pro glu ala lys arg met pro cys ala glu asp tyr leu ser val val leu asn gln leu cys val leu his glu lys thr pro val ser

CCT GAA GCA AAA AGA ATG CCC TGT GCA GAA GAC TAT CTA TCC GTG GTC CTG AAC CAG TTA TGT GTG TTG CAT GAG AAA ACG CCA GTA AGT (1520)

471                     476 477       480                               490                         500

asp arg val thr lys cys cys thr glu ser leu val asn arg arg pro cys phe ser ala leu glu val asp glu thr tyr val pro lys

GAC AGA GTC ACC AAA TGC TGC ACA GAA TCC TTG GTG AAC AGG CGA CCA TGC TTT TCA GCT CTG GAA GTC GAT GAA ACA TAC GTT CCC AAA (1610)

501                             510          514                         520                         530

glu phe asn ala glu thr phe thr phe his ala asp ile cys thr leu ser glu lys glu arg gln ile lys lys gln thr ala leu val

GAG TTT AAT GCT GAA ACA TTC ACC TTC CAT GCA GAT ATA TGC ACA CTT TCT GAG AAG GAG AGA CAA ATC AAG AAA CAA ACT GCA CTT GTT (1700)

531                   540                          550              558 559 560

glu leu val lys his lys pro lys ala thr lys glu gln leu lys ala val met asp asp phe ala ala phe val glu lys cys cys lys
GAG CTC GTG AAA CAC AAG CCC AAG GCA ACA AAA GAG CAA CTG AAA GCT GTT ATG GAT GAT TTC GCT GCT TTT GTA GAG AAG TGC TGC AAG (1790)

561                   567     570                        580

ala asp asp lys glu thr cys phe ala glu glu gly lys lys leu val ala ala ser gln ala ala leu gly leu ter         ter
GCT GAC GAT AAG GAG ACC TGC TTT GCC GAG GAG GGT AAA AAA CTT GTT GCT GCA AGT CAA GCT GCC TTA GGC TTA TAA CATCACATTTAAAAG (1883)

            ter    ter
CATCTCAGCCTACCATGAGAATAAGAGAAAGAAAATGAAGATCAAAAGCTTATTCATCTGTTTTTCTTTTTCGTTGGTGTAAAGCCAACACCCTGTCTAAAAAACATAAATTTCTTTAA (2002)

TCATTTTGCCTCTTTTCTCTGTGCTTCAATTAATAAAAAAATGGAAAGAATCTAA..... 20 .....AA (2078)

9.   Nucleotide sequence coding for the pre pro human serum albumin, said nucleotide sequence is as follows:

```
          pre                    -10              -18
          Met lys trp val glu phe ile ser leu leu phe leu phe ser
          ATG AAG TGG GTA ACC TTT ATT TCC CTT CTT TTT CTC TTT AGC (30)

     20            10         1        -1    -6   pro
     ser ala tyr ser arg gly val phe arg asp ala his lys ser glu val ala his lys asp leu arg phe lys
     TCG GCT TAT TCC AGG GGT GTG TTT CGT CGA GAT GCA CAC AAG AGT GAG GTT GCT GCT CAT CGG TTT AAA GAT TTG GGA GAA GAA AAT TTC AAA (170)

  21          30       34        40              50
  ala leu val leu ile ala phe ala gln tyr leu gln cys pro phe glu asp his val lys leu val asn glu val thr glu phe ala
  GCC TTG GTG TTG ATT GCC TTT GCT CAG TAT CTT CAG TGT CCA TTT GAA GAT CAT GTT AAA TTA GTG AAT GAA GTA ACT GAA TTT GCA (260)

  51  53      60    62        70       75         80
  lys thr cys val ala asp glu ser ala glu asn cys asp lys ser leu his thr leu phe gly asp lys leu cys thr val ala thr leu
  AAA ACA TGT GTT GCT GAT GAG TCA GCT GAA AAT TGT GAC AAA TCA CTT CAT ACC CTT TTT CGA GAC AAA TTA TGC ACA GTT GCA ACT CTT (350)

  81          90  91            100 101            110
  arg glu thr tyr gly glu met ala asp cys cys ala lys gln glu pro gly arg asn asn glu gln his lys asp asn pro
  CGT GAA ACC TAT GGT GAA ATG GCT GAT TGC TGC GCA AAG CAG GAG CCT GGG AGA AAT AAT GAA CAA CAC AAA GAC AAC CCA (440)

  111        120    124          130             140
  asn leu pro arg leu val arg pro glu val asp val met cys thr ala phe his asp asn glu gln thr phe leu lys lys tyr leu try
  AAC CTC CCC CGA TTG GTG AGA CCA GAG GTT GAT GTG ATG TGC ACT GCT TTT CAT GAC AAT GAA CAG ACA TTT TTG AAA AAA TAC TTA TAT (530)

  141        150              160      168 169 170
  glu ile ala arg arg his pro tyr phe tyr ala pro glu leu leu phe phe ala lys arg tyr lys ala ala phe thr glu cys cys gln
  GAA ATT GCC AGA AGA CAT CCT TAT TTT TAT GCC CCT GAA CTC CTT TTC TTT GCT AAA AGG TAT AAA GCT GCT TTT ACA GAA TGT TGC CAA (620)

  171    177     180              190             200
  ala ala asp lys ala ala cys leu leu pro lys leu asp glu leu arg asp glu gly lys ala ser ser ala lys gln arg leu lys cys
  GCT GCT GAT AAA GCT GCC TGC CTG TTG CCA AAG CTC GAT GAA CTC CGG GAT GAA GGG AAG GCT TCG TCT GCC AAA CAG AGA CTC AAG TGT (710)

  201        210             220              230
  ala ser leu gln lys phe gly glu arg ala phe lys ala trp ala val ala arg leu ser gln arg phe pro lys ala glu phe ala glu
  GCC AGT CTC CAA AAA TTT GGA GAA AGA GCT TTC AAA GCA TGG GCA GTA GCT CGC CTG AGC CAG AGA TTT CCC AAA GCT GAG TTT GCA GAA (800)
```

```
231
val ser lys leu val thr asp leu val his gly asp leu leu glu cys ala asp asp arg ala asp leu
GTT TCC AAG TTA GTG ACA GAT CTT CAT GGA GAT CTG CTT GAA TGT GCT GAT GAC AGG GCG GAC CTT (890)

261
ala lys tyr ile cys glu asn gln asp ser ile ser ser lys leu lys glu lys pro leu leu ser his cys ile
GCC AAG TAT ATC TGT GAA AAT CAA GAT TCG ATC TCC AGT AAA CTG AAG GAA AAA CCT CTG TTG TCC CAC TGC ATT (980)

291
ala glu val glu asn asp glu met pro ala asp leu pro ser leu ala ala asp phe ala asp val cys lys asn tyr ala
GCC GAA GTG GAA AAT GAT GAG ATG CCT GCA GAT CTG CCT TCA CTA GCT GCT GAT TTT GCT GAT GTT TGC AAA AAC TAT GCT (1070)

321
glu ala lys asp val phe leu gly met phe leu tyr glu tyr ala arg arg his pro asp tyr ser val val leu leu arg leu ala
GAG GCA AAG GAT GTC TTC TTG GGC ATG TTT TTG TAT GAA TAT GCA AGA AGG CAT CCT GAT TAC TCT GTC GTG CTG CTG AGA CTT GCC (1160)

351
lys thr tyr glu thr thr leu glu lys cys ala ala ala asp pro his glu tyr ala lys val phe asp glu phe lys pro leu
AAG ACA TAT GAA ACC ACT CTA GAG AAG TGC GCC GCA GAG GAT CCT CAT GAA TAT GCC AAA GTG TTC GAT GAG TTC AAA CCT CCT (1250)

381
val glu glu pro gln asn leu ile lys gln asn cys glu leu phe glu gln leu gly glu tyr lys phe gln asn ala leu leu val arg
GTG GAA GAG CCT CAG AAT TTA ATC AAA CAA AAT TGT GAG CTT TTT GAG CAG CTT GGA GAG TAC AAA TTT CAG AAT GCG CTG CTG GTT CGT (1340)

411
tyr thr lys lys val pro gln val ser pro thr leu val glu val ser arg asn leu gly lys val gly ser lys cys cys lys his
TAC ACC AAG AAA GTA CCC CAA GTG TCA CCA ACT CTT GTA GAG GTC TCA AGA AAC CTA GGA AAA GTG GGC AGC AAA TGT TGT AAA CAT (1430)

441
pro glu ala lys arg met pro cys ala glu asp tyr leu ser val val leu asn gln leu cys val leu his glu lys thr pro val ser
CCT GAA GCA AAA AGA ATG CCC TGT GCA GAA GAC TAT CTA TCC GTG GTC CTG AAC CAG CTT TGT GTG TTG CAT GAG AAG ACG CCA GTA AGT (1520)

471
asp arg val thr lys cys cys thr glu ser leu val asn arg arg pro cys phe ser ala leu glu val asp glu thr tyr val pro lys
GAC AGA GTC ACC AAA TGC TGC ACA GAA TCC TTG GTG AAC AGG CGA CCA TGC TTT TCA GCT CTT GAA GTC GAT GAA ACA TAC GTT CCC AAA (1610)

501
glu phe asn ala glu thr phe thr phe his ala asp ile cys thr leu ser glu lys glu arg gln ile lys lys gln thr ala leu val val
GAG TTT AAT GCT GAA ACA TTC ACC TTC CAT GCA GAT ATA TGC ACA CTT TCT GAG AAG GAG AGA CAA ATC AAG AAA CAA ACT GCA CTT GTT (1700)
```

531
glu leu val lys his lys pro lys ala thr lys glu gln leu lys ala val met asp asp phe ala ala phe val glu lys cys cys lys
GAG CTC GTG AAA CAC AAG CCC AAG GCA ACA AAA GAG CAA CTG AAA GCT GTT ATG GAT GAT TTC GCT GCT TTT GTA GAG AAG TGC TGC AAG (1790)

561
ala asp asp lys glu thr cys phe ala glu glu gly lys lys leu val ala ala ser gln ala ala leu gly leu ter          ter
GCT GAC GAT AAG GAG ACC TGC TTT GCC GAG GAG GGT AAA AAA CTT GTT GCT GCA AGT CAA GCT GCC TTA GGC TTA TAA CATCACATTTAAAAG (1883)

        ter   ter
CATCTCAGCCTACCATGAGAATAAGAGAAAGAAAATGAAGATCAAAAGCTTATTCATCTGTTTTTCTTTTTCGTTGGTGTAAAGCCAACACCCTGTCTAAAAAAACATAAATTTCTTTAA (2002)

TCATTTTGCCTCTTTTCTCTGTGCTTCAATTAATAAAAAAATGGAAAGAATCTAA..... 20 .....AA (2078)

10.    A nucleotide sequence according to any of claims 6 to 9, in essentially pure form.

11.    A DNA transfer vector comprising a nucleotide sequence as defined in claim 5.

12.    A DNA transfer vector according to claim 11, transferred to and replicated in a micro-organism.

13.    A DNA transfer vector according to claim 12, which is a plasmid.

14.    A DNA transfer vector according to claim 13, wherein the plasmid is pBR322 or YEp6.

15.    A process for preparing human serum albumin, which comprises culturing a micro-organism according to claim 5.

16.    A DNA transfer vector according to any of claims 12 to 14, or a process according to claim 15, wherein the micro-organism is a bacterium or yeast.

17.    A vector or process according to claim 16, wherein the bacterium or yeast is E. coli or Saccharomyces cerevisiae.

# Restriction Endonuclease Map of Human Serum Albumin cDNA Clones

## pHA36

**Kilobases**

0  .1  .2  .3  .4  .5  .6  .7  .8  .9  1.0  1.1  1.2  1.3  1.4  1.5  1.6  1.7  1.8  1.9  2.0

## pHA206